# EUROPEAN PATENT APPLICATION

(11) **EP 1 430 848 A1**
(43) Date of publication of application: **23.06.2004**
(21) Application number: 03078555.4
(22) Date of filing: 12.11.2003
(51) Int. Cl.: A61B 18/02, A61B 18/12

(54) **Cold tip RF/ultrasonic ablation catheter**

(30) Priority: 11.12.2002 US 318370
(71) Applicant: Cryocor, Inc., San Diego, California 92121 (US)
(72) Inventor: Sherman, Marshall, Cardiff by the Sea, California 92007 (US); Ryba, Eric, San Diego, California 92122 (US); Ayers, Gregory M., Rancho Santa Fe, California 92067 (US)
(74) Representative: Gates, Marie Christina Esther

(57) **Abstract**

A catheter for ablating internal tissue using radiofrequency (rf) or ultrasonic energy includes a mechanism to cool the catheter's distal tip. The catheter also includes either an rf electrode or an ultrasonic transducer positioned at the tip to direct energy into the internal tissue for tissue ablation. With the distal tip of the catheter positioned adjacent the target tissue, a refrigerant is introduced into the catheter and allowed to expand near the catheter's distal tip. During expansion, the fluid refrigerant transitions from a liquid state to a gaseous state. Latent heat absorbed during the phase transition cools the tip of the catheter to a temperature sufficient to prevent tissue charring and coagulum formation during ablation. In another implementation, the catheter tip is cooled to a temperature sufficient to freeze tissue and create an ice-ball at the catheter tip that stabilizes the tip relative to the target tissue.

## Description

### FIELD OF THE INVENTION

The present invention pertains generally to ablation catheters. More particularly, the present invention pertains to catheters for ablating internal tissue with radio-frequency (rf) or ultrasonic energy. The present invention is particularly, but not exclusively, useful as an ablation catheter that is internally cooled to prevent the formation of coagulum in the patient's bloodstream.

### BACKGROUND OF THE INVENTION

Atrial fibrillation is an irregular heart rhythm that adversely affects approximately 2.5 million people in the United States. It is believed that at least one-third of all atrial fibrillation originates near the ostium of the pulmonary veins, and that the optimal treatment technique is to ablate these focal areas through the creation of circumferential or linear lesions around the ostia of the pulmonary veins. More specifically, the goal is to ablate tissue to form a conduction block to thereby prohibit the transmission of irregular electrical signals that can cause an arrhythmia. To be effective, the conduction block must completely block irregular signals and this often requires the formation of a relatively deep, uniform lesion.

Platforms that use rf and ultrasonic energy to ablate tissue generate heat that can complicate the ablation procedure. For example, in rf ablation, ohmic heating occurs when current passes through tissue due to the resistance of the tissue. In general, it requires more power to ablate deeper lesions with a corresponding increase in the amount of heat that is generated. The heat that is generated during an ablation procedure can lead to tissue charring and the formation of coagulum in the patient's bloodstream that, in turn, can cause a stroke. Additionally, excessive heat can lead to a stenosis at the ablation site. Heretofore, methods for cooling the catheter tip using saline solutions have been disclosed. In one case, saline is introduced into the patient's vasculature upstream of the ablation site using a showerhead-type nozzle to cool the catheter tip. In another method, a closed loop of cooled saline solution is passed through the catheter. Unfortunately, neither of these methods provide adequate cooling to dissipate the relatively large quantity of heat that is generated when ablating relatively deep lesions.

Another factor that must be considered when ablating internal tissue using rf and ultrasonic energy is the stability of the catheter tip relative to the target tissue. During ablation, movements of the patient such as breathing and heartbeats can cause the tip of the catheter to move or bounce. Failure to prevent these movements of the catheter relative to the target tissue can disrupt the flow of energy to the tissue and cause non-uniform ablation. This disruption of energy flow often results in an ineffective conduction block.

In light of the above it is an object of the present invention to provide a catheter for safely ablating internal tissue. It is yet another object of the present invention to provide a catheter for ablating tissue with rf or ultrasonic energy that is cooled to prevent the formation of coagulum in the patient's bloodstream. Yet another object of the present invention is to provide a catheter that can ablate relatively deep lesions with relatively high power levels of rf or ultrasonic energy without tissue charring or the formation of coagulum. It is still another object of the present invention to provide a catheter that can be stabilized in position relative to target tissue during ablation. Yet another object of the present invention is to provide a catheter for ablation of internal tissue which is easy to use, relatively simple to manufacture, and comparatively cost effective.

### SUMMARY OF THE PREFERRED EMBODIMENTS

In a first aspect of the present invention, a catheter for ablating internal tissue includes a catheter body that is tubular-shaped and is formed with a lumen. The catheter body has an open proximal end and a distal end that is closed by a tip. Together, the tip and the catheter body form a chamber at the distal end of the catheter body. The catheter further includes a supply tube which has a proximal end and a distal end, and is formed with an orifice at its distal end. Operationally, the supply tube is positioned inside the lumen of the catheter body with the orifice of the supply tube positioned inside the chamber adjacent the tip of the catheter body. In a preferred embodiment of the present invention, the supply tube is positioned inside the lumen of the catheter body to establish a return line between the inner surface of the catheter body and the outer surface of the supply tube.

A fluid supply unit is provided to introduce a fluid refrigerant into the proximal end of the supply tube. The fluid refrigerant then traverses through the lumen of the supply tube and exits through the orifice at the distal end of the tube. As the fluid refrigerant exits through the orifice, it expands into the chamber to cool the catheter tip. In a particular embodiment of the present invention, the fluid refrigerant transitions from a liquid state to a gaseous state as it passes through the orifice. Heat absorbed by the refrigerant during this phase transition (i.e. latent heat) cools the tip of the catheter. After expansion, the gaseous fluid refrigerant passes through the return line and exits the catheter at the proximal end of the catheter body.

The catheter also includes a mechanism for directing energy into the internal tissue from the catheter tip to ablate internal target tissue. In a first embodiment of the present invention, radiofrequency (rf) current is used to ablate tissue. In this embodiment, the catheter includes an rf electrode that is positioned at the distal end of the catheter. A return electrode is positioned in contact with the patient and at an extracorporeal location. An rf generator is electrically wired to each electrode to pass an rf current from the catheter tip, through the internal tissue and to the return electrode. In another embodiment of the present invention, ultrasonic energy is used to ablate internal target tissue. In this embodiment, an ultrasonic transducer is positioned at the distal end of the catheter to pass ultrasonic energy through the internal tissue.

In operation, the distal end of the catheter is first inserted into the vasculature of a patient and advanced until the catheter tip is positioned adjacent the internal tissue to be ablated. In a first implementation of the present invention, rf current is used to ablate the internal tissue and the fluid refrigerant is used to cool the rf electrode and catheter tip to prevent tissue charring and the formation of coagulum in the patient's vasculature. In this implementation, a fluid refrigerant having an ambient pressure boiling point above zero degrees Celsius is used to cool the tip and prevent coagulum formation.

In another implementation of the present invention, ultrasonic energy is used to ablate the internal tissue and the fluid refrigerant is used to cool the ultrasonic transducer and catheter tip to prevent tissue charring and the formation of coagulum in the patient's vasculature. To prevent coagulum formation, a fluid refrigerant having an ambient pressure boiling point above zero degrees Celsius is used to cool the tip.

In yet another implementation, a fluid refrigerant is used to cool the catheter tip to a temperature just below zero degrees Celsius. In this case, tissue in contact with the cryotip freezes to the cryotip. The consequence here is that the cryotip can be fixed (i.e. stick) at a specific point against the tissue that is to be ablated.

In still another implementation of the present invention, the fluid refrigerant is used to cool the catheter tip (including the rf electrode/ultrasonic transducer) to very low temperatures sufficient to freeze tissue and blood. For example, a fluid refrigerant such as Nitrous Oxide having an ambient pressure boiling point below minus eighty-eight degrees Celsius (-88°C) can be used to freeze tissue and blood. In this implementation, a so-called "ice-ball" is formed at the catheter tip that can be used to stabilize the catheter tip relative to the target tissue. Once formed, the "ice ball" acts as a "virtual electrode." More specifically, the "ice ball" provides no effective impedance to the rf/ultrasonic energy that is radiated from the catheter tip. Accordingly, the surface area of the radiating energy source is effectively increased to be the surface of the "ice ball." This results in the beneficial consequence that the rf/ultrasonic power can be increased without adversely increasing the density of the current passing through tissue undergoing ablation. Thus, with the formation of the "ice ball," higher levels of power can be used to ablate tissue without a corresponding increase in coagulum formation or tissue charring.

In yet another implementation of the present invention, the fluid refrigerant is used to cool the catheter tip to temperatures sufficient to cryoablate tissue to a predetermined depth, d₁. Thereafter, the rf generator/ultrasonic transducer can be activated to pass energy through the cryoablated tissue to ablate underlying tissue to a second depth d₂, with d₂ > d₁-.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Fig. 1 is a perspective view of a patient showing a catheter for ablating internal tissue positioned in the patient's vasculature;
Fig. 2 is a cross-sectional view of the distal end portion of the catheter shown in Fig. 1 as seen along the line 2-2 in Fig. 1;
Fig. 3 is a cross-sectional view of the distal end portion of a catheter as in Fig. 2 showing an alternate embodiment of an ablation catheter in which ultrasonic energy is used for tissue ablation; and
Fig. 4 is a schematic view showing the distal portion of an ablation catheter positioned against internal tissue.

### DESCRIPTION OF THE PREFERRED EMBODIMENTS

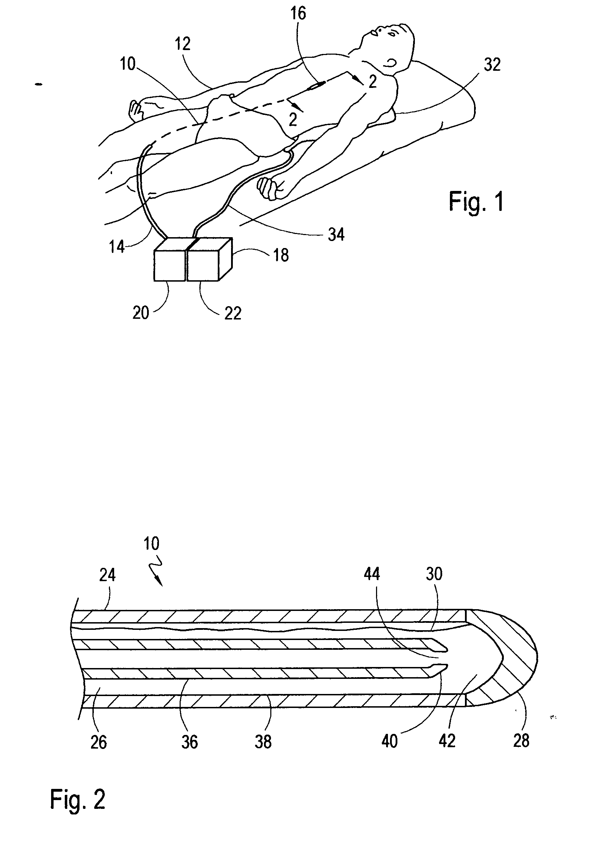

Referring initially to Fig. 1, a catheter 10 for ablating internal target tissue of a patient 12 is shown. As shown, the catheter 10 extends from a proximal end 14 that remains outside the patient's body during the procedure to a distal end 16. From Fig. 1 it can be seen that the distal end 16 of the catheter 10 has been inserted into the patient 12 through a vein such as the femoral vein and advanced through the patient's vasculature until the distal end 16 is positioned in the upper body of the patient 12. Fig. 1 further shows that the proximal end 14 of the catheter 10 is connected to a catheter control console 18 that is positioned at an extracorporeal location and includes an rf generator 20 and a fluid refrigerant supply unit 22.

With cross-reference now to Figs. 1 and 2, it can be seen that the catheter 10 includes a catheter body 24 that is tubular shaped and defines a lumen 26. An rf electrode 28 is mounted on the catheter body 24 at the distal end 16 of the catheter 10. As further shown, wire 30 extends through lumen 26 establishing an electrical connection between rf electrode 28 and rf generator 20. A return electrode 32 in contact with the exterior of the patient 12 is provided to receive current from rf electrode 28 after the current has passed through internal tissue of the patient 12. The return electrode 32 is also electrically connected to the rf generator 20 via wire 34, as shown.

Continuing with cross-reference to Figs. 1 and 2, it can be seen that the catheter 10 further includes a refrigerant supply tube 36 that is connected to the refrigerant supply unit 22, and extends through lumen 26 of catheter body 24. A return line 38 is thereby established between the catheter body 24 and supply tube 36 to return refrigerant to the refrigerant supply unit 22. The used refrigerant may be recycled or may be disposed of separately from the supply unit 22. For use in the catheter 10, refrigerant supply unit 22 may include gas storage bottles, compressors, precoolers or any other elements necessary for providing refrigerant under pressure. Also, refrigerant supply unit 22 may also include elements to control the pressure of the refrigerant such as valves and pressure regulators.

As best seen in Fig. 2, the distal end 40 of the supply tube 36 is positioned proximal to the rf electrode 28 to establish an expansion chamber 42 therebetween. Supply tube 36 is further formed with an orifice 44 to allow fluid refrigerant to flow from the supply tube 36 and into the chamber 42. It is to be appreciated from Fig. 2 that fluid refrigerant exiting through orifice 44 expands as it enters the chamber 42. In one implementation of the catheter 10, the fluid refrigerant is delivered to the orifice 44 in a liquid state for transition to a gaseous state as it passes through the orifice 44. The phase transition causes the refrigerant to absorb heat (i.e. latent heat) which in turn cools the rf electrode 28. Gaseous refrigerant is evacuated from the chamber 42 via suction on the return line 38 and exits the catheter 10 at the proximal end 14.

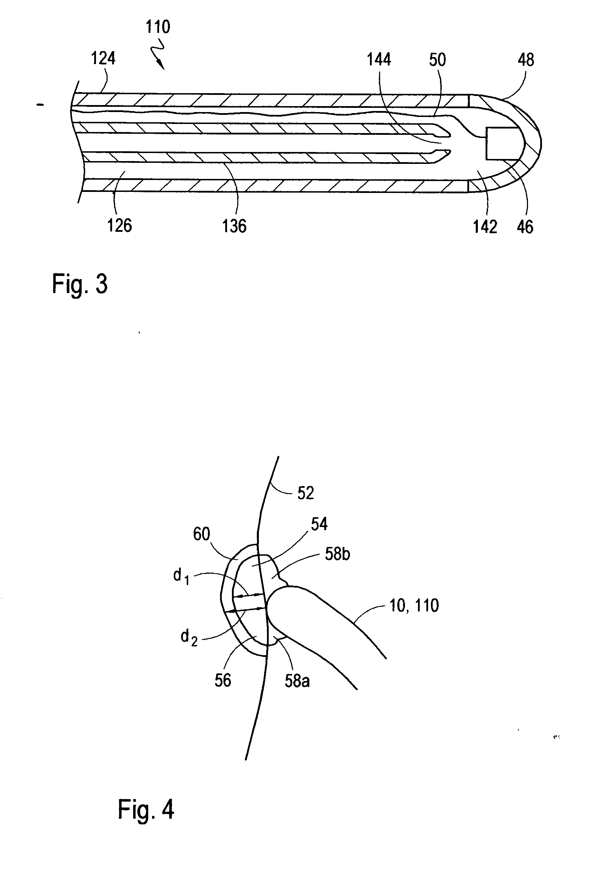

Fig. 3 shows another embodiment of a catheter (designated 110) for ablating internal tissue. In this embodiment, the catheter 110 includes an ultrasonic transducer 46 for generating ultrasonic energy to ablate internal tissue. As shown, the ultrasonic transducer 46 is positioned at the distal end of chamber 142 that is formed by the distal tip 48 and catheter body 124. Wire 50 extends through the lumen 126 of the catheter body 124 to connect the ultrasonic transducer 46 to an electric current source that is located at an extracorporeal location. Supply tube 136 is positioned, proximal to the ultrasonic transducer 46 and is formed with orifice 144 to allow fluid refrigerant flowing from the supply tube 136 to expand into the chamber 142 and cool the ultrasonic transducer 46 and distal tip 48.

The operation of the catheters 10, 110 can best be appreciated with reference to Fig. 4. As shown, the distal end of the catheter 10, 110 is positioned in the vasculature of the patient and in contact with the surface 52 of internal tissue 54 to be ablated. In a first implementation of the present invention, rf current (catheter 10) or ultrasonic energy (catheter 110) is used to ablate the internal tissue and the fluid refrigerant is used to cool the rf electrode 28 / ultrasonic transducer 46 (see Figs. 2 and 3) and catheter tip to prevent tissue charring and the formation of coagulum in the patient's vasculature. In this implementation, a fluid refrigerant having an ambient pressure boiling point above zero degrees Celsius is expanded into the chamber 42, 142 to cool the tip of the catheter 10, 110 and prevent tissue charring and coagulum formation.

In another implementation, the cooling system of the catheter 10, 110 can be used to create a so-called "ice-ball" at the catheter tip that includes a first layer 56 of frozen target tissue that extends a distance d₁ from the surface 52 and frozen blood 58a,b. To create the "ice ball", a fluid refrigerant such as Nitrous Oxide having an ambient pressure boiling point below minus eighty-eight degrees Celsius (-88°C) is expanded in the chamber 42, 142 (See Figs. 2 and 3). As shown in Fig. 4, the frozen blood 58a,b attaches to catheter 10, 110 to the surface 52 to stabilize the catheter tip relative to the target tissue 54. Tissue in the first layer 56 can be cryoablated using a refrigerant such as nitrous oxide in the catheter 10, 110. Alternatively, tissue in the first layer 56 can be ablated using rf/ultrasonic energy with cooling to prevent coagulum formation as described above.

Once the first layer 56 is ablated and the "ice ball" is established, a second layer 60 that extends to a depth of d₂ from the surface 52 can be ablated using rf/ultrasonic energy. During ablation of the second layer 60, the "ice ball" that is created acts as a "virtual electrode." Stated another way, the "ice ball" provides no effective impedance to the rf/ultrasonic energy that is radiated from the catheter tip. Accordingly, the surface area of the radiating electrode is effectively increased to be the surface of the "ice ball." This results in the beneficial consequence that the rf/ultrasonic power can be increased without adversely increasing current density. Hence, the probability of creating coagulum is reduced. Typically, rf power in the range up to 100 watts can be used during ablation of the second layer 60.

While the particular cold tip rf/ultrasonic ablation catheter as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as described in the appended claims.

## Claims

1. An ablation catheter for ablating internal tissue, said ablation catheter comprising:
a catheter body formed with a lumen, said catheter body having an open proximal end and a closed distal end, said closed distal end defining a catheter tip for said catheter and forming a chamber in said catheter body;
a means for directing energy into the internal tissue from said catheter tip to ablate the internal tissue;
a supply tube having a proximal end and a distal end with an orifice formed at said distal end, said supply tube being positioned in said lumen of said catheter body with said orifice positioned in said chamber adjacent said catheter tip; and
a means for introducing a fluid refrigerant into said supply tube through said proximal end thereof for expansion of the fluid refrigerant into said chamber through said orifice to cool said catheter tip during ablation of said tissue.

2. A catheter as recited in claim 1 wherein said means for directing energy into the internal tissue comprises an electrode for passing an electrical current into the tissue for receipt by a return electrode.

3. A catheter as recited in claim 2 wherein said electrode passes said electrical current to a return electrode positioned at an extracorporeal location.

4. A catheter as recited in claim 2 or 3 wherein said electrical current is a radio-frequency (rf) current.

5. A catheter as recited in any one of claims 1 to 4 wherein said means for directing energy into the internal tissue comprises an ultrasonic transducer for passing ultrasonic energy into the tissue.

6. A catheter as recited in any one of claims 1 to 5 wherein said supply tube is positioned in said lumen of said catheter body to establish a return line therebetween.

7. A catheter as recited in any one of claims 1 to 6 wherein at least a portion of said fluid refrigerant transitions from a first phase to a second phase prior to exiting said chamber.

8. A catheter as recited in any one of claims 1 to 7 wherein at least a portion of said fluid refrigerant enters said chamber in the liquid phase and transitions to a gas phase in said chamber to cool said catheter tip.

9. A catheter as recited in any one of claims 1 to 8 wherein said fluid refrigerant has an ambient pressure boiling point above zero degrees Celsius.

10. A catheter as recited in any one of claims 1 to 8 wherein said fluid refrigerant has an ambient pressure boiling point below minus eighty-eight degrees Celsius (-88°C).

11. A method for ablating the internal tissue of a patient, which comprises the steps of:
providing a catheter having a catheter body formed with a lumen, said catheter body having an open proximal end and a closed distal end, said closed distal end defining a catheter tip for said catheter and forming a chamber in said catheter body, an electrode for directing radio-frequency (rf) current from said catheter tip, and a supply tube having a proximal end and a distal end with an orifice formed at said distal end, said supply tube being positioned in said lumen of said catheter body with said orifice positioned in said chamber adjacent said catheter tip of said catheter;
introducing a fluid refrigerant into said supply tube through said proximal end thereof for expansion of the fluid refrigerant into said chamber through said orifice to cool said catheter tip; and
activating an rf generator to direct rf current from said electrode and through the tissue to ablate the tissue.

12. A method for ablating the internal tissue of a patient, the internal tissue formed with a surface, said method comprising the steps of:
positioning the tip of a catheter against the surface of the internal tissue;
cooling said catheter tip to a temperature sufficient to cryoablate the internal tissue to a first depth from the surface of the internal tissue, d₁; and thereafter
passing radiofrequency current through the internal tissue from said catheter tip to ablate the internal tissue to a second depth from the surface of the internal tissue, d₂, wherein said second depth d₂ is larger than said first depth, d₁ (d₂ > d₁).
